# EUROPEAN PATENT APPLICATION

(11) **EP 3 181 135 A1**
(43) Date of publication of application: **21.06.2017**
(21) Application number: 15826950.6
(22) Date of filing: 31.07.2015
(51) Int. Cl.: A61K 31/7004, A61K 45/00, A61P 3/04, A61P 43/00

(54) **ADIPONECTIN SECRETION REGULATOR**

(30) Priority: 31.07.2014 JP 2014156767
(71) Applicant: The University of Tokyo, Tokyo 113-8654 (JP); Yaizu Suisankagaku Industry Co., Ltd., Yaizu-shi, Shizuoka 425-8570 (JP)
(72) Inventor: OHARA, Kazuyuki, Tokyo 113-8654 (JP); USHIO, Hideki, Tokyo 113-8654 (JP); SATONE, Hina, Tokyo 113-8654 (JP); HATTORI, Takeshi, Yaizu-shi Shizuoka 425-8570 (JP); UENO, Tomoya, Yaizu-shi Shizuoka 425-8570 (JP)
(74) Representative: Denison, Christopher Marcus
(86) International application number: PCT/JP2015/071774
(87) International publication number: WO 2016/017796

(57) **Abstract**

To provide an adiponectin secretion regulator whereby the effect of adiponectin can be efficiently expressed while the adverse effect of appetite increase associated with adiponectin is avoided, and to provide a food/drink product, a functional food product, a cosmetic, a pharmaceutical, and an animal feed having such adiponectin secretion regulating effects.

In the present invention, fucose or a precursor thereof is used as an active ingredient of the adiponectin secretion regulator. A food/drink product, a functional food product, a cosmetic, a pharmaceutical, or an animal feed having adiponectin secretion regulating effects is obtained by compounding a specific amount of fucose or a precursor thereof.

## Description

### TECHNICAL FIELD

The present invention relates to an adiponectin secretion regulator containing fucose as an active ingredient.

### BACKGROUND ART

Adiponectin is a protein secreted from adipocytes, and is a hormone that circulates at a relatively high concentration in the blood. The physiological functions thereof are in maintaining energy homeostasis and in sugar/fat metabolism and the like, and adiponectin is reported to be involved in, e.g., increased insulin resistance or fatty acid combustion via activation of AMP-activated protein kinase (AMPK) (Non-patent Document 1). Adiponectin circulates through the blood not only in trimers, but is also present as hexamers or larger multimers (e.g., 12- to 18-mers), and it is clear that the fatty acid combustion or insulin resistance increasing effect thereof is high particularly for multimers (Non-patent Document 2). It is also clear that trimeric or hexameric adiponectin passes through the blood-brain barrier and acts on appetite centers in the hypothalamus to increase appetite (Non-patent Document 3).

Active substances/ingredients for promoting production or regulating secretion of adiponectin have been discovered in the past and utilized in pharmaceuticals, cosmetics, food/drink products, animal feeds, and the like. (Patent Documents 1-3, for example.)

### [Prior Art Documents]

### [Patent Documents]

[Patent Document 1] Japanese Patent No. 4785140
[Patent Document 2] Japanese Patent No. 5004153
[Patent Document 3] Japanese Patent No. 5499415

### [Non-patent Documents]

[Non-patent Document 1] Yamauchi, T., Kamon, J., Minokoshi, Y., Ito, Y., Waki, H., Uchida, S., Yamashita, S., Noda, M., Kita, S., Ueki, K., Eto, K., Akanuma, Y., Froguel, P., Foufelle, F., Ferre, P., Carling, D., Kimura, S., Nagai, R., Kahn, B. B., and Kadowaki, T. "Adiponectin stimulates glucose utilization and fatty-acid oxidation by activating AMP-activated protein kinase". Nat. Med. (2002) 8, pp. 1288-1295.
[Non-patent Document 2] Pajvani, U. B., Hawkins, M., Combs, T. P., Rajala, M. W., Doebber, T., Berger, J. P., Wagner, J. A., Wu, M., Knopps, A., Xiang, A. H., Utzschneider, K. M., Kahn, S. E., Olefsky, J. M., Buchanan, T. A., Scherer, P. E. "Complex distribution, not absolute amount of adiponectin, correlates with thiazolidinedione-mediated improvement in insulin sensitivity". J. Biol. Chem. (2004) 279, pp. 12152-62.
[Non-patent Document 3] Kubota, N., Yano, W., Kubota, T., Yamauchi, T., Itoh, S., Kumagai, H., Kozono, H., Takamoto, I., Okamoto, S., Shiuchi, T., Suzuki, R., Satoh, H., Tsuchida, A., Moroi, M., Sugi, K., Noda, T., Ebinuma, H., Ueta, Y., Kondo, T., Araki, E., Ezaki, O., Nagai, R., Tobe, K., Terauchi, Y., Ueki, K., Minokoshi, Y., Kadowaki, T. "Adiponectin stimulates AMP-activated protein kinase in the hypothalamus and increases food intake". Cell Metab. (2007) Jul; 6(1) pp. 55-68.

### DISCLOSURE OF THE INVENTION

### [Problems to Be Solved by the Invention]

However, because of the appetite increasing effects associated with adiponectin, as described above, merely increasing the blood concentration of adiponectin or the amount of production thereof from adipocytes is extremely inconvenient in such cases as when avoidance of overeating is desired to promote health.

An object of the present invention is therefore to provide an adiponectin secretion regulator whereby the effect of adiponectin can be efficiently expressed while the adverse effect of appetite increase associated with adiponectin is avoided, and to provide a food/drink product, a functional food product, a cosmetic, a pharmaceutical, and an animal feed having such adiponectin secretion regulating effects.

### [Means to Solve the Problems]

As a result of concentrated studies aimed at achieving the abovementioned objects, the inventors discovered that fucose has the effects of reducing generation of adiponectin monomers, trimers, and hexamers, and of promoting multimerization of adiponectin, and perfected the present invention.

Specifically, the present invention comprises the features described below.
[1] An adiponectin secretion regulator characterized by containing fucose or a precursor thereof as an active ingredient.
[2] The adiponectin secretion regulator according to [1], for increasing a relative amount of adiponectin multimers with respect to a total amount of adiponectin monomers, trimers, and hexamers.
[3] The adiponectin secretion regulator according to [1] or [2], for reducing a total amount of adiponectin monomers, trimers, and hexamers.
[4] The adiponectin secretion regulator according to any of [1] through [3], for appetite suppression.
[5] The adiponectin secretion regulator according to any of [1] through [3], for fat combustion.
[6] The adiponectin secretion regulator according to any of [1] through [3], for anti-obesity.
[7] The adiponectin secretion regulator according to any of [1] through [3], for improving insulin resistance.
[8] The adiponectin secretion regulator according to any of [1] through [3], for administration together with another adiponectin secretion regulator having the effect of increasing blood concentration of adiponectin.
[9] A food/drink product containing fucose or a precursor thereof in a ratio of 0.0001 to 50% by mass in terms of the fucose content and having adiponectin secretion regulating effects.
[10] A functional food product containing fucose or a precursor thereof in a ratio of 0.0001 to 100% by mass in terms of the fucose content and having adiponectin secretion regulating effects.
[11] A cosmetic containing fucose or a precursor thereof in a ratio of 0.0001 to 50% by mass in terms of the fucose content and having adiponectin secretion regulating effects.
[12] A pharmaceutical containing fucose or a precursor thereof in a ratio of 0.0001 to 100% by mass in terms of the fucose content and having adiponectin secretion regulating effects.
[13] An animal feed containing fucose or a precursor thereof in a ratio of 0.0001 to 50% by mass in terms of the fucose content and having adiponectin secretion regulating effects.

### [Advantageous Effects of the Invention]

Through the present invention, because fucose has the effects of reducing generation of adiponectin monomers, trimers, and hexamers and of promoting multimerization of adiponectin, the useful effect of adiponectin can be efficiently obtained while the adverse effect of appetite increase associated with adiponectin is avoided.

### BRIEF DESCRIPTION OF THE DRAWINGS

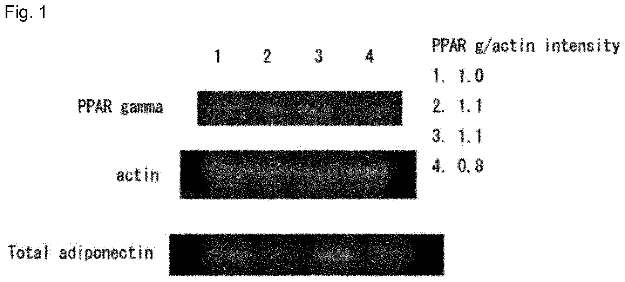

FIG. 1 is a diagram illustrating the results of investigating the effect of fucose on generation of adiponectin by adipocytes, and illustrates the results of investigating the expression levels of the nuclear-receptor-type transcription factor PPAR-gamma, actin as an internal standard protein, and adiponectin by a Western blotting method after culturing for 24 hours in a serum-free medium including 0.1 w/v% of glucose (Lane 1), 0.1 w/v% of glucose + 0.1 w/v% of fucose (Lane 2), 0.45 w/v% of glucose (Lane 3), or 0.45 w/v% of glucose + 0.1 w/v% of fucose (Lane 4).

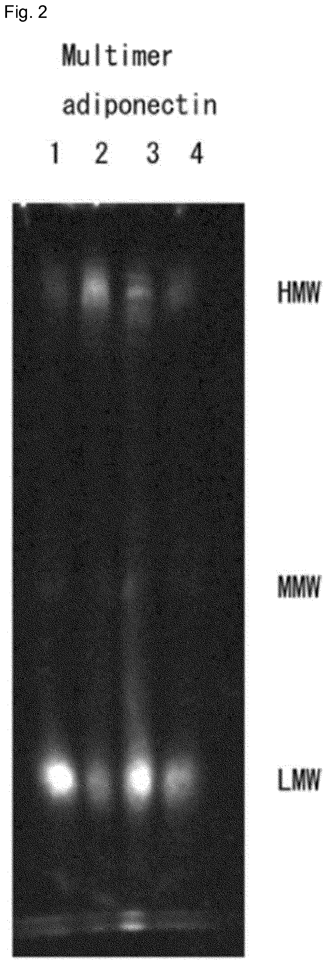

FIG. 2 is a diagram illustrating the results of investigating the effect of fucose on generation of adiponectin by adipocytes, and illustrates the results of investigating the expression level and formation state of adiponectin multimers by a Western blotting method after culturing for 24 hours in a serum-free medium including 0.1 w/v% of glucose (Lane 1), 0.1 w/v% of glucose + 0.1 w/v% of fucose (Lane 2), 0.45 w/v% of glucose (Lane 3), or 0.45 w/v% of glucose + 0.1 w/v% of fucose (Lane 4).

### BEST MODE FOR CARRYING OUT THE INVENTION

The adiponectin secretion regulator according to the present invention has fucose as an active ingredient. The present invention can be applied not only for humans, but for animals as well. Insofar as the effects of fucose can be expressed, a fucose precursor that necessarily generates fucose by biolysis when administered to a human or animal can also be used as the active ingredient. Examples of fucose precursors include fungal cell walls, fungal secreted polysaccharides, seaweed extract, potato (*Solanum tuberosum*) extract, cassava (*Manihot esculenta*) extract, kiwi fruit (*Actinidia deliciosa*) extract, thale cress (*Arabidopsis thaliana*) extract, soybean (*Glycine max*) extract, winged bean (*Psophocarpus tetragonolobus*) extract, water shield (*Brasenia schreberi*) extract, ribgrass (*Plantago lanceolata*) extract, garden cress (*Lepidium sativum*) extract, hollyhock (*Alcea rosea*) extract, kapok (*Ceiba pentandra*) extract, Chinese licorice (*Glycyrrhiza uralensis*) extract, lupin (*Lupinus luteus*) extract, tragacanth (*Astragalus gummifer*) extract, fucoidan, ascophyllan, pectin, mucin, blood, fucose glycosides, fucose derivatives, milk oligosaccharides, tragacanth, and the like.

The adiponectin secretion regulator according to the present invention preferably contains the abovementioned active ingredient in a ratio of 0.0001 to 100% by mass, more preferably 0.0005 to 70% by mass, and more preferably 0.001 to 50% by mass, in terms of the fucose content. When the content of the active ingredient is less than the abovementioned range, the amount of fucose when the adiponectin secretion regulator is administered to the human or animal becomes insufficient, and the effect of the adiponectin secretion regulator tends to decrease.

The mode of formulation of the adiponectin secretion regulator according to the present invention is not particularly limited; the abovementioned active ingredient may be used alone or compounded, as needed, with, for example, a pharmaceutically allowable substrate, carrier, or the like. The adiponectin secretion regulator may be in the form of, e.g., a tablet, a powder, granules, a capsule, a liquid, a lozenge, a chewable tablet, a candy, a jelly, an injection, an inhaled agent, an ointment, or the like by a publicly known method.

The mode of administration of the adiponectin secretion regulator according to the present invention is not particularly limited; the adiponectin secretion regulator may be administered orally, intravenously, locally in the brain, intraperitoneally, by inhalation, transnasally, by topical application, or by another method, for example.

The administered amount of the adiponectin secretion regulator according to the present invention is appropriately set in accordance with such factors as the mode of administration, the patient state or health state of the human or animal to which the present invention is applied, but is not particularly limited. In the typical case of oral administration, the administered amount is preferably about 0.1 to 20,000 mg per day for an adult in terms of fucose, and more preferably 0.1 to 10,000 mg.

As described below, the active ingredient of the adiponectin secretion regulator according to the present invention has the effect of increasing the relative amount of adiponectin multimers with respect to the total amount of adiponectin monomers, trimers, and hexamers, and of reducing the total amount of adiponectin monomers, trimers, and hexamers, and is therefore suitable for such purposes as appetite suppression, fat combustion, anti-obesity, and improvement of insulin resistance, for example.

The adiponectin secretion regulator according to the present invention may also be administered together with another adiponectin secretion regulator having the effect of increasing blood concentration of adiponectin, for example. Although the adverse effect of increased appetite is generally produced by adiponectin monomers, trimers, and hexamers when the blood concentration of adiponectin is increased, as described below, the active ingredient of the adiponectin secretion regulator according to the present invention has the effect of increasing the relative amount of adiponectin multimers with respect to the total amount of adiponectin monomers, trimers, and hexamers, and of reducing the total amount of adiponectin monomers, trimers, and hexamers, and therefore also suppresses the adverse effect of increased appetite associated with the other adiponectin secretion regulator, and makes it possible to efficiently obtain the advantageous effects of adiponectin overall. Examples of the other adiponectin secretion regulator include thiazolidine derivatives, gamma-oryzanol and compositions thereof, cinnamic acid phenethyl esters, curcumin, and the like.

Meanwhile, another aspect of the present invention provides a food/drink product, a functional food product, a cosmetic, a pharmaceutical, and an animal feed containing fucose or a precursor thereof and having adiponectin secretion regulating effects.

The abovementioned food/drink product preferably contains fucose or a precursor thereof in a ratio of 0.0001 to 50% by mass, more preferably 0.0005 to 30% by mass, and more preferably 0.001 to 15% by mass, in terms of the fucose content. When the amount of fucose or precursor thereof is less than the abovementioned range, the amount of fucose ingested becomes insufficient, and the effect of the adiponectin secretion regulation therefore tends to deteriorate. Examples of food/drink products include: (1) soft drinks, carbonated beverages, fruit juice beverages, vegetable juices, lactic acid bacteria beverages, milk beverages, soy milk, mineral water, tea beverages, coffee beverages, sports drinks, alcoholic beverages, jelly beverages, and other beverages; (2) tomato puree, canned mushrooms, dehydrated vegetables, pickles, and other processed vegetable products; (3) dehydrated fruit, jam, fruit puree, canned fruits, and other processed fruit products; (4) curry powder, wasabi, ginger, spice blends, powdered seasonings, and other cooking spices; (5) pasta, udon, soba, ramen, macaroni, and other noodles (including raw noodles and dry noodles); (6) bread, sweet baked goods, dressed bread, doughnuts, and other breads; (7) pregelatinized rice, oatmeal, wheat bran, batter powder, and the like; (8) baked confectioneries, biscuits, rice confectioneries, candy, chocolate, chewing gum, snack confectioneries, frozen desserts, candied confectioneries, Japanese sweets, Western sweets, semiperishable sweets, pudding, ice cream, and other sweets; (9) adzuki beans, tofu, natto, soy flour, tofu skin, cooked beans, peanuts, and other beans products; (10) honey and royal jelly processed food; (11) ham, sausage, bacon, and other meat products; (12) yogurt, pudding, condensed milk, cheese, fermented milk, butter, ice cream, and other dairy products; (13) processed egg products, (14) dried fish, boiled fish paste, tubular fish paste, fish sausage, and other processed fish, or dried seaweed, kelp, tsukudani, and other processed seaweed, or cod roe, herring roe, salmon roe, dried mullet roe, and other processed fish eggs (15) instant bouillon, soy sauce, vinegar, mirin, consomme base, Chinese food base, concentrated soup stock, dressings, mayonnaise, ketchup, miso, and other seasonings, or salad oil, sesame oil, linoleic oil, diacylglycerol, safflower oil, and other edible oils and fats; and (16) soups (including powdered and liquid soups) and other cuisine and semi-prepared foods, or side dishes, retort foods, chilled foods, semi-prepared foods (e.g., takikomi rice mix and crabmeat omelet mix), and the like.

The abovementioned functional food product preferably contains fucose or a precursor thereof in a ratio of 0.0001 to 100% by mass, more preferably 0.0005 to 70% by mass, and more preferably 0.001 to 50% by mass, in terms of the fucose content. When the amount of fucose or precursor thereof is less than the abovementioned range, the amount of fucose ingested becomes insufficient, and the effect of the adiponectin secretion regulation therefore tends to deteriorate. Examples of functional food products include health foods, health drinks, supplements, nutritional supplementary foods, health-promoting functional foods, foods for specified health uses, foods for which the functionality thereof is permitted to be displayed, food additive materials, and the like.

The form of the functional food product is not particularly limited; a substrate, carrier, additive, nutrient component, or the like allowed in foods, for example, may be combined with the functional food product as needed, and the functional food product may be configured in the form of, e.g., a tablet, a powder, granules, a capsule, a liquid, a candy, a jelly, or the like by a publicly known method.

The abovementioned cosmetic preferably contains fucose or a precursor thereof in a ratio of 0.0001 to 50% by mass, more preferably 0.0005 to 30% by mass, and more preferably 0.001 to 15% by mass, in terms of the fucose content. When the amount of fucose or precursor thereof is less than the abovementioned range, the amount of fucose applied becomes insufficient, and the effect of the adiponectin secretion regulation therefore tends to deteriorate.

The form of the cosmetic is not particularly limited; a substrate, carrier, additive, moisturizing component, or the like allowed in cosmetics, for example, may be combined with the cosmetic as needed, and the cosmetic may be configured in the form of, e.g., a cream, a lotion, an atomized spray, a foamy spray, a soap, a gel, a face mask, or the like by a publicly known method.

The abovementioned pharmaceutical preferably contains fucose or a precursor thereof in a ratio of 0.0001 to 100% by mass, more preferably 0.0001 to 70% by mass, and more preferably 0.0001 to 50% by mass, in terms of the fucose content. When the amount of fucose or precursor thereof is less than the abovementioned range, the amount of fucose ingested becomes insufficient, and the effect of the adiponectin secretion regulation therefore tends to deteriorate.

The form of the pharmaceutical is not particularly limited; a substrate, carrier, additive, efficacy-supplementing component, or the like allowed in pharmaceuticals, for example, may be combined with the pharmaceutical as needed, and the pharmaceutical may be configured in the form of, e.g., a tablet, a powder, granules, a capsule, a liquid, a lozenge, a chewable tablet, a candy, a jelly, an injection, an inhaled agent, an ointment, or the like by a publicly known method.

The abovementioned animal feed preferably contains fucose or a precursor thereof in a ratio of 0.0001 to 50% by mass, more preferably 0.0005 to 30% by mass, and more preferably 0.001 to 15% by mass, in terms of the fucose content. When the amount of fucose or precursor thereof is less than the abovementioned range, the amount of fucose ingested becomes insufficient, and the effect of the adiponectin secretion regulation therefore tends to deteriorate. Examples of animals herein include dogs, cats, birds, and other pets; cows, pigs, chickens, horses, sheep, goats, and other livestock; tuna, yellowtail, eel, sea bream, pufferfish, and other cultured fish; and the like.

The form of the animal feed is not particularly limited; the animal feed may be solid, semi-solid, powdered, liquid, or the like. A substrate, carrier, excipient for feed, or the like allowed in an animal feed, for example, as well as other feed materials and the like may be combined with the animal feed as needed. Examples of other feed materials include grain flour, sugar, salt, oils and fats, vitamins, amino acids, polyphenols, nucleic acids, animal protein, plant protein, meat extracts, fish extracts, yeast extracts, taste agents, dyes, lactic acid bacteria, antibiotics, hormones, and the like.

Through the knowledge obtained through the present invention, there are provided not only an adiponectin secretion regulator containing fucose or a precursor thereof as an active ingredient, but also, *inter alia,* a method for regulating secretion of adiponectin by administering fucose or a precursor thereof to a human or animal, or a use of fucose or a precursor thereof for regulating secretion of adiponectin.

In the abovementioned method, there is also provided a method for increasing the relative amount of adiponectin multimers with respect to the total amount of adiponectin monomers, trimers, and hexamers, a method for reducing the total amount of adiponectin monomers, trimers, and hexamers, a method for suppressing appetite, a method for fat combustion, an anti-obesity method, a method for improving insulin resistance, or a method for administering the adiponectin secretion regulator together with another adiponectin secretion regulator having the effect of increasing blood concentration of adiponectin.

In the abovementioned use, there is also provided a use for increasing the relative amount of adiponectin multimers with respect to the total amount of adiponectin monomers, trimers, and hexamers, a use for reducing the total amount of adiponectin monomers, trimers, and hexamers, a use for suppressing appetite, a use for fat combustion, a use for anti-obesity, a use for improving insulin resistance, or a use for administering the adiponectin secretion regulator together with another adiponectin secretion regulator having the effect of increasing blood concentration of adiponectin.

### Examples

The present invention will be more specifically described below by way of examples, but these examples are not limiting of the scope of the present invention.

### <Test Example 1>

Mouse 3T3-L1 fibroblasts in a confluent state were induced with insulin to differentiate into adipocytes. The cells were then cultured for 48 hours in a DMEM medium containing 10% FBS. The cells were then cultured for 24 hours in a serum-free DMEM medium including L-fucose and/or D-glucose at the concentrations for each test section indicated in Table 1.

**[Table 1]**

| | Concentration in serum-free DMEM medium |
|---|---|
| Test Section 1 (Lane 1) | 0.1 w/v% glucose |
| Test Section 2 (Lane 2) | 0.1 w/v% glucose + 0.1 w/v% fucose |
| Test Section 3 (Lane 3) | 0.45 w/v% glucose |
| Test Section 4 (Lane 4) | 0.45 w/v% glucose + 0.1 w/v% fucose |

After culturing, the culture supernatant was collected and mixed with an equal amount of reducing SDS-PAGE (polyacrylamide gel electrophoresis) loading buffer and held for 10 minutes at 100°C, a 5-10-µL sample for each lane was subjected to SDS-PAGE, and the nuclear-receptor-type transcription factor PPAR-gamma, actin as an internal standard protein, and adiponectin were detected by a Western blotting method using specific antibodies for each in accordance with the usual method. A near-infrared fluorescence imager ("Odyssey Fc," manufactured by LI-COR, Inc.) was used for the detection, and the relative expression levels of each protein were compared. The results are indicated in FIG. 1.

As indicated in FIG. 1, in test sections 2 and 4 in which L-fucose was added to the medium at a concentration of 0.1 w/v%, a tendency for the expression level of adiponectin to decrease was recognized in both the 0.1 w/v% and the 0.45 w/v% glucose concentration sections in comparison with test sections 1 and 3 in which fucose was not added. At this time, when the expression level of the nuclear-receptor-type transcription factor PPAR-gamma regulating the generation of adiponectin was examined, in test section 2 in which D-glucose was added to the medium at a concentration of 0.1 w/v% and L-fucose was added to the medium at a concentration of 0.1 w/v%, almost no change in the expression level of PPAR-gamma was recognized in comparison with test section 1 in which D-glucose was added to the medium at a concentration of 0.1 w/v%. Meanwhile, in test section 4 in which D-glucose was added to the medium at a concentration of 0.45 w/v% and L-fucose was added to the medium at a concentration of 0.1 w/v%, a slight decrease in the expression level of PPAR-gamma was recognized in comparison with test section 3 in which D-glucose was added to the medium at a concentration of 0.45 w/v%.

### <Test Example 2>

The cell culture supernatant in the same test sections as in Test Example 1 was collected, equivalent samples for each test section were subjected to non-reducing SDS-PAGE, and adiponectin monomers appearing at a molecular weight position of approximately 30 kDa, trimers appearing at a position of approximately 100 kDa, hexamers appearing at a position of approximately 200 kDa, and multimers appearing at higher molecular weight positions were detected by a Western blotting method using specific antibodies for adiponectin. A near-infrared fluorescence imager ("Odyssey Fc," manufactured by LI-COR, Inc.) was used for the detection, and the various multimerization states were compared. The results are indicated in FIG. 2.

As indicated in FIG. 2, in test sections 2 and 4 in which L-fucose was added to the medium at a concentration of 0.1 w/v%, it was apparent that the incidence of monomers, trimers, and hexamers was reduced, and multimers were increased in both the 0.1 w/v% and the 0.45 w/v% glucose concentration sections in comparison with test sections 1 and 3 in which fucose was not added. This tendency was more pronounced in the low-glucose-concentration sections (0.1 w/v%).

It is apparent from the results of Test Examples 1 and 2 that fucose has the effect of promoting multimerization of adiponectin secreted by adipocytes. The present invention is thus useful for suppressing the adverse effect of appetite increase associated with adiponectin monomers, trimers, and hexamers, and for efficiently expressing the effects of adiponectin multimers.

## Claims

1. An adiponectin secretion regulator **characterized by** containing fucose or a precursor thereof as an active ingredient.

2. The adiponectin secretion regulator according to claim 1, for increasing a relative amount of adiponectin multimers with respect to a total amount of adiponectin monomers, trimers, and hexamers.

3. The adiponectin secretion regulator according to claim 1 or 2, for reducing a total amount of adiponectin monomers, trimers, and hexamers.

4. The adiponectin secretion regulator according to any of claims 1 through 3, for appetite suppression.

5. The adiponectin secretion regulator according to any of claims 1 through 3, for fat combustion.

6. The adiponectin secretion regulator according to any of claims 1 through 3, for anti-obesity.

7. The adiponectin secretion regulator according to any of claims 1 through 3, for improving insulin resistance.

8. The adiponectin secretion regulator according to any of claims 1 through 3, for administration together with another adiponectin secretion regulator having the effect of increasing blood concentration of adiponectin.

9. A food/drink product containing fucose or a precursor thereof in a ratio of 0.0001 to 50% by mass in terms of the fucose content and having adiponectin secretion regulating effects.

10. A functional food product containing fucose or a precursor thereof in a ratio of 0.0001 to 100% by mass in terms of the fucose content and having adiponectin secretion regulating effects.

11. A cosmetic containing fucose or a precursor thereof in a ratio of 0.0001 to 50% by mass in terms of the fucose content and having adiponectin secretion regulating effects.

12. A pharmaceutical containing fucose or a precursor thereof in a ratio of 0.0001 to 100% by mass in terms of the fucose content and having adiponectin secretion regulating effects.

13. An animal feed containing fucose or a precursor thereof in a ratio of 0.0001 to 50% by mass in terms of the fucose content and having adiponectin secretion regulating effects.
